# FASCICULE DE BREVET EUROPEEN

(11) **EP 1 230 223 B1**
(45) Date de publication et mention de la délivrance du brevet: **25.02.2004**
(21) Numéro de dépôt: 00976109.9
(22) Date de dépôt: 06.11.2000
(51) Int. Cl.: C07D 235/18, C07D 235/20, A61K 31/4184, A61K 31/7088, A61K 48/00, A61K 49/00, G01N 33/52, C12N 15/87

(54) **DERIVES D'OLIGOBENZIMIDAZOLES ET LEUR UTILISATION COMME AGENTS DE TRANSFECTION D'ADN**
OLIGOBENZIMIDAZOLDERIVATE UND IHRE VERWENDUNG ALS DNA-TRANSFEKTIONSMITTEL
OLIGOBENZIMIDAZOLE DERIVATIVES AND THEIR USE AS DNA TRANSFECTION AGENTS

(30) Priorité: 05.11.1999 FR 9913934; 05.01.2000 US 174648 P
(43) Date de publication de la demande: 14.08.2002
(73) Titulaire: Aventis Pharma S.A., 92165 Antony Cédex (FR)
(72) Inventeur: SCHERMAN, Daniel, F-75012 Paris (FR); BESSODES, Michel, F-94800 Villejuif (FR); PITARD, Bruno, F-44300 Nantes (FR); SOTO, Javier, E-36201 Vigo (ES); BYK, Gérardo, 55513 Qyriat Ono (IL)
(74) Mandataire: Bouvet, Philippe
(86) Numéro de dépôt international: PCT/FR2000/003087
(87) Numéro de publication internationale: WO 2001/032630

(56) Documents cités:
- EP-A- 0 653 491
- GB-A- 2 144 542
- CHEMICAL ABSTRACTS, vol. 121, no. 11, 12 septembre 1994 (1994-09-12) Columbus, Ohio, US; abstract no. 124572z, WONG S S C ET AL: "Transcriptional regulation of differentiation, selective toxicity and ATGCAAAT binding of bisbenzimidazole derivatives in human melanoma cells" page 20; XP002143428 cité dans la demande & BIOCHEM. PHARMACOL., vol. 47, no. 5, 1994, pages 827-37,
- SOTO J ET AL: "Non-electrostatic complexes with DNA: towards novel synthetic gene delivery systems" BIOORGANIC & MEDICINAL CHEMISTRY LETTERS, vol. 10, no. 9, mai 2000 (2000-05), pages 911-914, XP004199041 ISSN: 0960-894X

## Description

La présente invention se rapporte à des dérivés d'oligobenzimidazoles capables de s'associer avec les acides nucléiques de formule générale (I) : 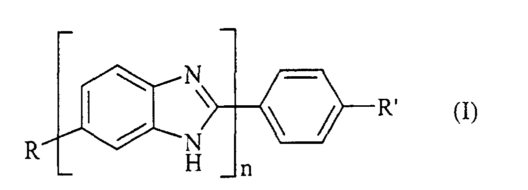 leurs sels, les compositions qui les contiennent et leurs utilisations, par exemple pour le transfert *in vitro, in vivo* ou *ex vivo* d'acides nucléiques dans les cellules ou la visualisation des acides nucléiques administrés par fluorescence.

Dans le brevet FR 1.519.964 ont été décrit les composés bis-benzimidazoliques et leurs sels de formule : 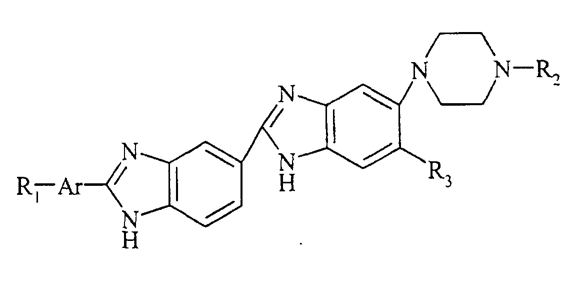 où Ar désigne un reste arylène, R₁ désigne un atome d'hydrogène ou d'halogène, un groupe hydroxy, un groupe alkyle ou alcoxy inférieur, un groupe mercapto ou alkylmercapto, un groupe alkylènedioxy ou nitro, un reste phényle ou un groupe aminé éventuellement porteur de substituants alkyliques, R₂ désigne l'hydrogène, un reste alkyle éventuellement substitué, un reste alcoxycarbonyle, carbamido, aryle ou aralkyle et R₃ désigne un atome d'halogène ou un reste alkyle inférieur. Ces composés sont décrits comme possédant une forte activité anthelmintique et bactériostatique contre des germes gram-positifs, et ils peuvent être également facilement caractérisés grâce à leur fluorescence verte typique (voir page 5 paragraphe 5 de FR 1.519.964).

Il a en outre été montré en particulier que l'un de ces dérivés bis-benzimidazoliques dénommé « Hoechst 33258 » pour lequel Ar-R₁ est le p-phénol, R₂ est un groupe méthyle et R₃ est H, est également un bon ligand du petit sillon de l'ADN, en plus d'être un fluorophore. Le « Hoechst 33258 » a donc été décrit comme étant utile pour la visualisation d'ADN nouvellement synthétisé et pour déterminer le nombre de paires de bases A-T présentes dans un échantillon d'ADN (voir F.G. Loontiens et al., *Biochemistry,* **1990**, 29, pp. 9029-9039).

EP 0 653 491 A1 décrit l'utilisation du composé « Hoechst 33258 » ou de son ether éthylique « Hoechst 33342 » pour l'introduction d'ADN étranger dans des cellules *in vitro.*

De nombreux composés similaires au « Hoechst 33258 » ont par la suite été synthétisés. Par exemple, un analogue pour lequel le groupe hydroxy du phénol terminal est placé en méta au lieu d'être en para, a été préparé dans le but d'introduire potentiellement une liaison hydrogène avec certains groupes fonctionnels de l'ADN (S.E. S. Ebrahimi et al., *Anti-Cancer Drug Design,* **1995,** 10, pp. 463-479). Il a été montré que cette légère différence structurelle par rapport au « Hoechst 33258 » n'introduisait pas de modification majeure des propriétés, bien que même une très légère modification de structure est susceptible d'altérer les propriétés de liaison à l'ADN (voir page 464 du même document). De tels dérivés sont décrits comme étant utiles en tant qu'outil biologique et comme médicaments dirigés directement vers le génome dans des cas de maladie virale ou de cancers.

Le composé « Hoechst 33258 » a également été modifié par l'introduction au niveau du substituant phénol terminal de différentes sortes de molécules de liaison (communément appelées « linker »), comme par exemple l'hexa(éthylène glycol), afin de pouvoir lier ce fluorophore de façon covalente à des oligo(déoxynucleotides), ce qui permet d'augmenter la stabilité du complexe d'hybridation formé et de suivre le succès de cette hybridation par mesure de la fluorescence (K. Wiederholt et al., *J. Am.* *Chem. Soc.,* **1996**, 118, pp. 7055-7062 ; Sharanabasava B. Rajur et al., *J. Org. Chem.,* **1997,** 62, pp. 523-529).

Des conjugués entre le « Hoechst 33258 » et du polyéthylène glycol (« PEG ») ont également été formés afin de permettre la séparation de fragment d'ADN amplifiés par réaction de polymérisation en chaîne (« PCR ») identiques en longueur mais différents en ce qui concerne leur composition basique, grâce aux propriétés de liaison du composé « Hoechst 33258 » à l'ADN (M. Müller et al., *Nucleic Acid Research,* **1997,** Vol. 25, N° 24, pp. 5125-5126).

Enfin, des analogues du « Hoechst 33258 » portant une chaîne alkyle contenant 5, 8 ou 12 atomes de carbone sur l'oxygène du phénol terminal ont également été synthétisés. Il a été montré que ces analogues lient le petit sillon de l'ADN et qu'il en résulte ainsi une inhibition de la transcription de certains gènes spécifiques des cellules cancéreuses. Il a aussi été montré que ces analogues induisent une toxicité sélective vis-à-vis des cellules du mélanome humain (S.S.C. Wong et al., *Biochemical Pharmacology,* **1994,** Vol. 47, N°5, pp. 827-837).

Par ailleurs, il est connu que les lipides cationiques sont des agents de transfection de l'ADN dans les cellules. En effet, du fait de leur charge globale positive, ils interagissent spontanément avec l'ADN globalement négatif, formant ainsi par interractions ioniques des complexes nucléolipidiques compactés capables de se fixer sur les membranes cellulaires, et permettent la libération intracellulaire de l'ADN. Toutefois, l'emploi de ces lipides cationiques en tant qu'agent de tranfection pose encore de nombreux problèmes, et leur efficacité reste à améliorer. Notamment, il a été observé que pour obtenir des complexes nucléolipidiques efficaces et stables, il est en général nécessaire que ces complexes soient fortement cationiques. Cependant, il serait souhaitable de pouvoir disposer de vecteurs non-cationiques ou moins cationiques de façon à former avec l'acide nucléique des particules globalement neutres ou négatives pour différentes raisons :
- les complexes formés entre l'acide nucléique et les vecteurs de transfert, du fait de leur charge globale positive, ont tendance à être captés par le système réticuloendothélial ce qui induit leur élimination,
- du fait de la charge globale positive des complexes formés, les protéines du plasma ont tendance à s'adsorber à leur surface, et il en résulte une perte du pouvoir de transfection,
- dans un contexte d'injection locale la présence d'une charge globale positive importante empêche la diffusion des complexes d'acides nucléiques en dehors du site d'administration, car les complexes s'adsorbent sur les matrices extracellulaires ; les complexes ne peuvent donc plus atteindre les cellules cibles, ce qui, par voie de conséquence, entraîne une diminution de l'efficacité de transfert par rapport à la quantité de complexes injectée,
- et enfin, les lipides ou polymères cationiques ont un effet inflammatoire, qui a été constaté à de nombreuses reprises.

Une alternative aux lipides cationiques pour le transfert d'acides nucléiques a ainsi été proposée dans la thèse de JS Rémy *(Synthèse et Utilisation in vitro de nouveaux vecteurs de transfert de gènes,* Jean-Serge REMY, Université Louis Pasteur de Strasbourg, soutenance du 13 avril 1994), dans le cadre de laquelle des oligopyrroles couplés à des chaînes grasses hydrocarbonées avaient été synthétisés afin de former des complexes avec l'ADN, grâce notamment aux propriétés de ligands du petit sillon de l'ADN des oligopyrroles peptidiques. Cependant, les dérivés lipidiques d'oligopyrrole synthétisés s'étaient avérées légèrement toxiques et n'avaient montré aucune efficacité en transfection. De tels vecteurs ne semblait donc pas être avantageux par rapport aux lipides cationiques.

Il a maintenant été montré que les dérivés d'oligobenzimidazole de formule générale (I) : 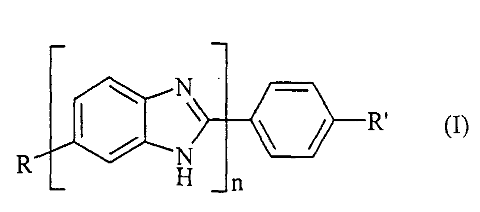 dans laquelle
- R représente un atome d'hydrogène, un radical carboxyle, alkyloxycarbonyle, carbamoyle, alkylcarbamoyle, un groupe pipérazinyle éventuellement substitué en position -4 par un alkyle contenant 1 à 4 atomes de carbone droit ou ramifié, ou bien R représente un groupe imidazolyle,
- n est un entier égal à 2, 3, 4 ou 5, et
- R' représente un groupe -O-R₃, -S-R₃, NHR₃, ou bien -O-CO-NH-R₃ et R₃ représente un groupe alkyle,
- ou bien R' représente un groupe -NR₄R₅ ou bien -O-CO-NR₄R₅ et R₄ et R₅, identiques ou différents, représentent chacun un groupe alkyle,
les radicaux alkyles mentionnés ci-dessus étant, sauf spécification contraire, droits ou ramifiés, éventuellement saturés, et contenant 12 à 22 atomes de carbone,
ainsi que leurs sels, manifestent des propriétés de liaison à l'ADN et des propriétés de fluorescence particulièrement intéressantes dans le cadre d'une administration d'ADN *in vitro, in vivo* ou *ex vivo* et sa visualisation.

En effet, les composés de formule générale (I) selon la présente invention constituent des dérivés du « Hoechst 33258 » couplés à une ou plusieurs chaînes grasses hydrocarbonées, et il a été montré que ces composés sont des ligands de l'ADN, et en particulier du petit sillon de l'ADN, mais que, contrairement aux lipides cationiques, ils ne compactent pas ledit ADN. Plus précisément, les dérivés d'oligobenzimidazole de formule générale (I) selon l'invention forment des liaisons hydrogènes non-ioniques avec l'ADN. Ils permettent ainsi de stabiliser l'ADN dans un contexte de production et/ou purification d'ADN. En outre, il a été également montré que les dérivés d'oligobenzimidazole selon l'invention conservent les mêmes propriétés de fluorescence que le « Hoechst 33258 » lorsqu'ils sont associés à l'ADN, ce qui permet la visualisation de ce dernier. Enfin, il a été mis en évidence que, contrairement aux oligopyrroles lipidiques, les dérivés d'oligobenzimidazole selon la présente invention permettent le transfert de l'ADN dans les cellules tout en le protégeant des dégradations causées par les endonucléases.

Selon une variante de l'invention, les dérivés d'oligobenzimidazole ont pour formule générale (II) : 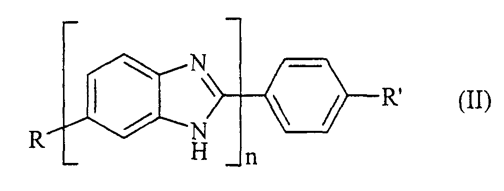 dans laquelle
- R représente un atome d'hydrogène ou un groupe pipérazinyle éventuellement substitué en position -4 par un alkyle contenant 1 à 4 atomes de carbone droit ou ramifié,
- n est un entier égal à 2 ou 3, et
- R' représente un groupe -OR₃ , NHR₃ ou -O-CO-NH-R₃ et R₃ représente un groupe alkyle,
- ou bien R' représente un groupe NR₄R₅ ou -O-CO-NR₄R₅ et R₄ et R₅, identiques ou différents, représentent chacun un groupe alkyle,
les radicaux alkyles mentionnés ci-dessus étant, sauf spécification contraire, droits ou ramifiés, éventuellement saturés, et contenant 12 à 22 atomes de carbone,
étant entendu que R' est différent de OR₃ avec R₃ représentant un substituant dodécyle lorsque R représente le méthyl-4 pipérazinyle et que n est égal à 2, ainsi que leurs sels.

Au sens de l'invention, les substituants alkyle droits ou ramifiés, éventuellement saturés, et contenant 12 à 22 atomes de carbone sont également appelés « chaînes grasses ». La ou les chaînes grasses peuvent contenir en particulier 12, 14, 16 ou 18 atomes de carbone. Il peut notamment s'agir des chaînes grasses (CH₂)₁₁CH₃, (CH₂)₁₃CH₃, (CH₂)₁₅CH₃ ou (CH₂)₁₇CH₃.

Les dérivés d'oligobenzimidazole de formule générale (I) peuvent être obtenus selon des méthodes analogues à celles décrites dans le brevet FR 1.519.964. C'est plus particulièrement le cas lorsqu'on veut obtenir des dérivés pour lesquels R représente un substituant pipérazinyle éventuellement substitué. En effet, on peut dans ce cas partir du produit « Hoechst 33258 » commercial et greffer sur le groupe hydroxy du phénol terminal le substituant R' tel que défini dans la formule générale (I) selon des méthodes classiques connues de l'homme du métier ou selon des méthodes analogues. Par ailleurs, les dérivés d'oligobenzimidazole de formule générale (I) peuvent être également obtenus soit par synthèse en phase solide, soit par synthèse en phase liquide.

### A - Préparation en phase liquide

A titre non-limitatif, on peut opérer de la façon suivante :
1) On condense sur le 1,2-diaminobenzène commercial du 3,4-dinitrobenzaldéhyde de façon à obtenir après cyclisation spontanée un dérivé nitré de formule générale (III) : 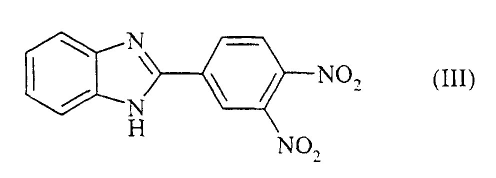 La condensation est effectuée dans le dioxane en présence de diiode. On opère de préférence à température comprise entre 10°C et 40°C pendant environ 24 heures.
   Le 3,4-dinitrobenzaldéhyde peut être obtenu de la façon suivante :
   a) L'acide 3,4-dinitrobenzoïque commercial est transformé en l'halogénure d'acyle correspondant selon les méthodes classiques connues de l'homme du métier pour obtenir un halogénure d'acyle à partir d'un acide ou selon des méthodes analogues. Par exemple, on opère en présence d'un réactif tel que le chlorure de thionyle, le trichlorure ou tribromure de phosphore, le pentachlorure ou pentabromure de phosphore à température comprise entre70°C et 90°C environ. Selon une autre méthode, on opère en présence de triphénylphosphine dans le tétrachlorométhane.
   b) L'halogénure de 3,4-dinitrobenzylcarbonyle est ensuite réduit en l'alcool correspondant selon les méthodes classiques connues de l'homme du métier pour obtenir un alcool à partir d'un halogénure d'acyle ou selon des méthodes analogues. A titre d'exemple, on peut opérer en présence de borohydrure de lithium dans un solvant convenable (par exemple du tétrahydrofurane) à très basse température, par exemple à - 78°C.
   c) L'alcool obtenu à l'étape précédente est enfin oxydé en 3,4-dinitrobenzaldéhyde selon les méthodes classiques connues de l'homme du métier pour obtenir un aldéhyde à partir d'un alcool ou selon des méthodes analogues. Par exemple, on peut utiliser comme agent oxydant l'oxyde de chrome et opérer en présence de chlorure de triméthylsilyle. On se place dans ce cas à température comprise entre 10°C et 40°C environ dans un solvant organique approprié comme par exemple la diméthylformamide, les solvants chlorés etc...
2) Le dérivé nitré de formule générale (III) est alors réduit de façon à obtenir un dérivé diaminé de formule générale (IV) : 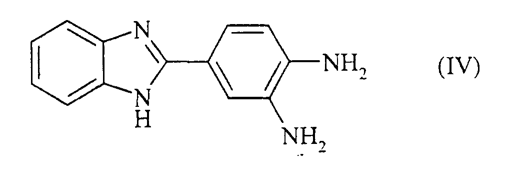 La réduction s'effectue selon les méthodes classiques, par exemple par hydrogénation catalytique en milieu acide en présence de nickel de Raney ou de palladium sur charbon, dans un alcool et à une température comprise entre 20 et 60°C. On peut utiliser à titre d'alcool du méthanol ou de l'éthanol. Une autre alternative consiste à opérer par action du chlorure stanneux en milieu aqueux acide à température comprise entre 20 et 100°C, ou encore par réduction par le fer en milieu aqueux acide et alcoolique à une température comprise entre 20 et 100°C. La solution aqueuse acide peut par exemple être une solution aqueuse d'acide chlorhydrique. La solution alcoolique peut par exemple être du méthanol ou de l'éthanol.
3) les étapes de condensation et de réduction telles que décrites en 1) et 2) sont répétées successivement n-2 fois de telle sorte que l'on obtient un dérivé diaminé de formule générale (V) : 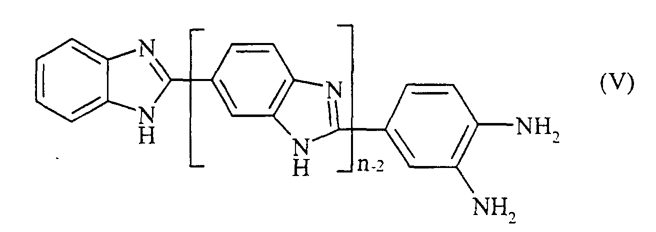 dans laquelle n représente un entier choisi parmi 2, 3, 4 ou 5.
4) On condense ensuite sur le dérivé diaminé de formule générale (V) obtenu précédemment un dérivé de nitrobenzaldéhyde de formule générale (VI) : 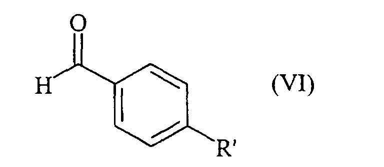 dans laquelle R' est tel que défini dans la formule générale (I),
   de façon à obtenir dérivé de formule générale (VII) : 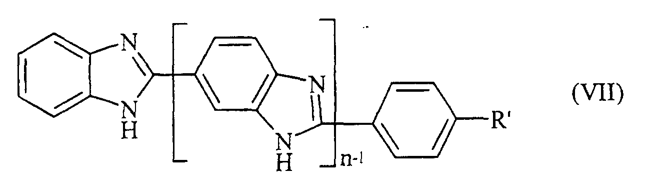 dans laquelle R' est tel que défini précédemment.
   De préférence, la condensation est effectuée dans le dioxane en présence de diiode. On opère préférentiellement à température comprise entre 10°C et 40°C pendant environ 24 heures. Selon une autre méthode, on opère en présence de dichloruredicyanoquinone (DDQ) dans un solvant convenable, par exemple la N,N-diméthylformamide, la N-méthylpyrrolidinone, la diméthylacétamide, l'acétonitrile, le dichlorométhane, le toluène, le benzène etc...
   Le dérivé benzaldéhyde de formule générale (VI) est soit commercial, soit il est obtenu :
   a) par alkylation du 4-hydroxybenzaldéhyde commercial selon les méthodes classiques connues de l'homme du métier ou selon des méthodes analogues lorsque R' représente un groupe OR₃,
   b) par réduction suivie d'une alkylation du 4-nitrobenzaldéhyde commercial selon les méthodes classiques connues de l'homme du métier ou selon des méthodes analogues lorsque R' représente un groupe NHR₃ ou, ou bien
   c) par addition nucléophile du dérivé acide COOH-NHR₃ ou COOH-NR₄R₅ sur du 4-hydroxybenzaldéhyde commercial selon les méthodes classiques connues de l'homme du métier ou selon des méthodes analogues lorsque R' représente un groupe -O-CO-NHR₃ ou bien -O-CO-NR₄R₅.
5) Dans le cas où l'on souhaite que les dérivés de formule générale (I) selon la présente invention portent un substituant R représentant un groupe pipérazinyle éventuellement substitué ou un groupe imidazolyle, alors on effectue la première étape du procédé décrit ci-avant à partir du 1,2-diaminobenzène substitué en position - 4 par le groupe R.

### B - Préparation en phase solide, première variante

On peut également préparer les dérivés d'oligobenzimidazole de formule générale (I) selon la présente invention en phase solide. C'est plus particulièrement le cas lorsque l'on souhaite que les dérivés de formule générale (I) selon la présente invention portent un substituant R représentant un groupe carboxyle, alkyloxycarbonyle, carbamoyle ou alkylcarbamoyle. Dans ce cas, on peut opérer comme suit :
1 ) Sur une résine classique de type résine de Wang substituée par un atome de brome ou d'iode ou par un groupe hydroxy, ou toute autre résine analogue convenable, on greffe l'acide 3,4-diaminobenzoïque commercial, de façon à obtenir la résine substituée de formule générale (VIII) : 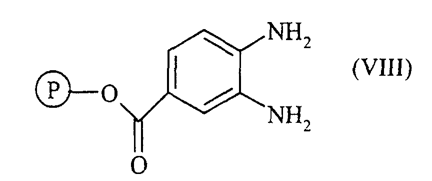 Lorsque la résine de départ est substituée par un atome d'halogène, le couplage est effectué en présence d'un sel de césium et d'une base non-nucléophile dans la N-éthyldiisopropylamine, dans un solvant aprotique convenable. A titre de base non nucléophile, on peut par exemple utiliser les amines tertiaires, du carbonate de calcium ou encore du dicarbonate de sodium. Encore plus préférentiellement, les bases utilisées sont des amines tertiaires, par exemple la triéthylamine (TEA) ou la N-éthyldiisopropylamine (DIEA). Les solvants convenables peuvent être choisis parmi la N-méthylpyrrolidinone ou la diméthylformamide.
2) Sur la résine substituée de formule générale (VIII) obtenue à l'étape précédente, on condense le 3,4-dinitrobenzaldéhyde de façon à obtenir après cyclisation spontanée un dérivé nitré de formule générale (IX) : 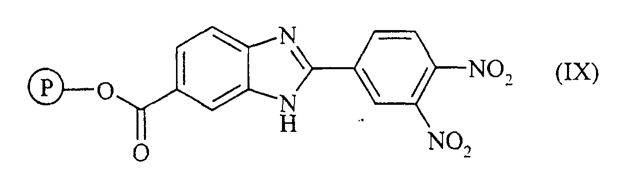 La condensation est effectuée dans le dioxane en présence de diiode. On opère de préférence à température comprise entre 10°C et 40°C pendant environ 24 heures. Le 3,4-dinitrobenzaldéhyde est obtenu de la même façon que décrit précédemment pour la préparation en phase liquide.
3) Le dérivé nitré de formule générale (IX) obtenu est alors réduit de façon à obtenir un dérivé diaminé de formule générale (X) : 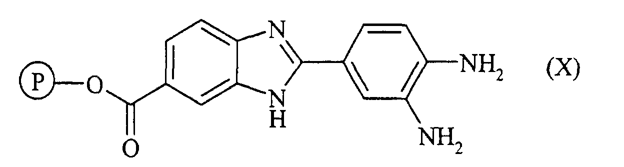 La réduction est effectuée de préférence en présence d'un acide de Lewis dans un solvant convenable. A titre d'acide de Lewis, on utilise par exemple le chlorure d'étain ou le chlorure de chrome. A titre de solvant convenable, on utilise par exemple la N,N-diméthylformamide ou la N-méthylpyrrolidinone
4) les étapes de condensation et de réduction telles que décrites ci-dessus en 2) et 3) sont répétées successivement encore n-2 fois de telle sorte que l'on obtient un dérivé diaminé de formule générale (XI) : 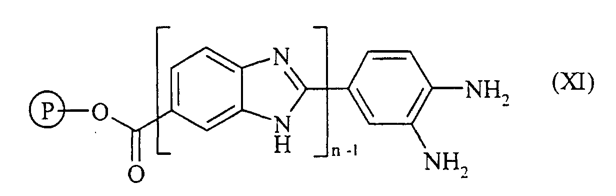 dans laquelle n représente un entier choisi parmi 2, 3, 4 ou 5.
5) On condense ensuite sur le dérivé diaminé de formule générale (XI) obtenu précédemment un dérivé de nitrobenzaldéhyde de formule générale (VI) : 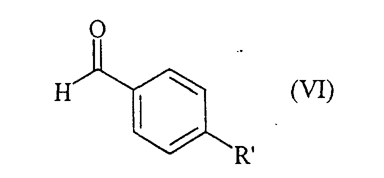 dans laquelle R' est tel que défini dans la formule générale (I), de façon à obtenir un dérivé de formule générale (XII) : 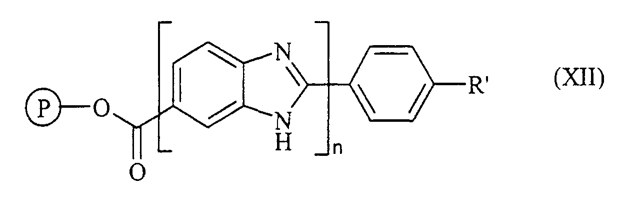 dans laquelle R' est tel que défini précédemment.
   De préférence, la condensation est effectuée dans le dioxane en présence de diiode.
   On opère préférentiellement à température comprise entre 10°C et 40°C pendant environ 24 heures. Selon une autre méthode, on opère en présence de dichloruredicyanoquinone (DDQ) dans un solvant convenable choisi parmi la N,N-diméthylformamide ou la N-méthylpyrrolidinone.
   Le dérivé benzaldéhyde de formule générale (VI) est soit commercial, soit il est obtenu comme indiqué précédemment pour la préparation en phase liquide.
6) Le dérivé obtenu à l'étape précédente est ensuite clivé de la résine, et on obtient ainsi l'acide de formule générale (XIII) : 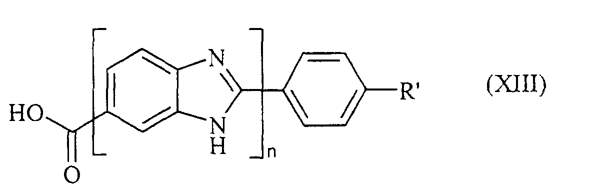 dans laquelle R' et n sont tels que définis précédemment.
   Le clivage de la résine est effectué selon les méthodes classiques connues de l'homme du métier ou selon toute autre méthode analogue. Par exemple, on opère en présence d'acide trifluoroacétique à température comprise entre 10°C et 50°C.
7) Pour obtenir les dérivés d'oligobenzimidazole de formule générale (I), on opère, selon la signification de R, de la façon suivante :
   a) lorsque R représente un radical alkyloxycarbonyle, on opère selon les méthodes classiques d'estérification connues de l'homme du métier qui n'altèrent pas le reste de la molécule, notamment par application ou adaptation des méthodes décrites dans Tetrahedron, 33, 683 (1977), Tetrahedron Letters, 4475 (1978) ou Bull. Soc. Chim. Japan, 40, 2380 (1967),
   b) lorsque R représente un radical carbamoyle ou alkylcarbamoyle, on opère selon les méthodes classiques de transformation des acides en amide connues de l'homme du métier et qui n'altèrent pas le reste de la molécule, par exemple par traitement avec de l'ammoniac ou avec une amine primaire convenable (pour R représentant un radical alkylcarbamoyle),

### C - Préparation en phase solide, seconde variante

Selon une autre variante, la synthèse en phase solide peut être effectuée comme suit :
1) Sur une résine classique de type résine de Wang substituée par un atome de brome ou d'iode ou par un groupe hydroxy, ou toute autre résine analogue convenable, on greffe l'acide 3-nitro-4-aminobenzoïque commercial, de façon à obtenir la résine substituée de formule générale (XIV) : 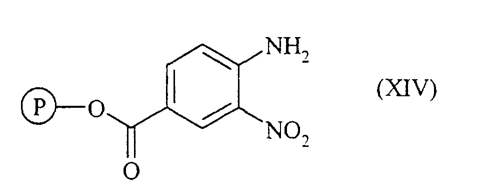 Lorsque la résine de départ est substituée par un atome d'halogène, le couplage est effectué en présence d'un sel de césium et d'une base non-nucléophile dans la N-éthyldiisopropylamine, dans un solvant aprotique convenable. A titre de base non nucléophile, on peut par exemple utiliser les amines tertiaires, du carbonate de calcium ou encore du dicarbonate de sodium. Encore plus préférentiellement, les bases utilisées sont des amines tertiaires, par exemple la triéthylamine (TEA) ou la N-éthyldiisopropylamine (DIEA). Les solvants convenables peuvent être choisis parmi la N-méthylpyrrolidinone ou la diméthylformamide.
2) A la résine substituée de formule générale (XIV) obtenue à l'étape précédente, on ajoute du chlorure de 4-fluoro-3-nitrobenzylcarbonyle et on obtient ainsi la résine substituée de formule générale (XV) suivante : 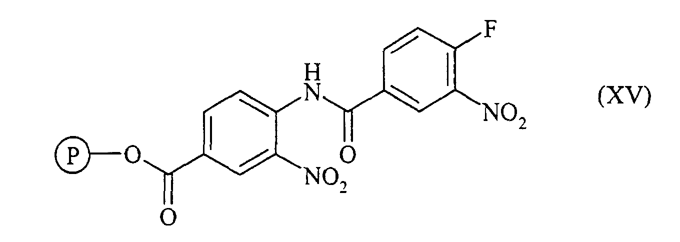 Le couplage est effectué selon les méthodes de couplage peptidique classiques (Bodanski M., Principles and Practices of Peptide Synthesis, Ed. Springe-Verlag) ou par toute méthode analogue connue de l'homme du métier. Notamment, la réaction s'effectue généralement en présence d'une base non-nucléophile dans des solvants aprotiques convenables, à température comprise entre 0 et 100°C, le pH étant ajusté entre 9 et 11.
   A titre d'exemple, le chloroforme, la diméthylformamide, la méthylpyrrolidone, l'acétonitrile, le dichlorométhane, le toluène ou le benzène peuvent être utilisés comme solvant.
   Les bases non-nucléophiles employées sont préférentiellement des amines tertiaires, du carbonate de calcium ou du dicarbonate de sodium. Encore plus préférentiellement, les bases utilisées sont des amines tertiaires comme par exemple la triéthylamine (TEA) ou le N-éthyldiisopropylamine.
   Avantageusement, le couplage peptidique est effectué entre 0 et 50°C, et de préférence entre 10 et 30°C.
   Le chlorure de 4-fluoro-3-nitrobenzylcarbonyle est obtenu a partir de l'acide commercial correspondant selon toute méthode connue de l'homme du métier pour obtenir un halogénure d'acyle à partir d'un acide. Par exemple, on peut opérer par action du chlorure de thionyle à température comprise entre 70°C et 90°C environ.
3) Puis, l'atome de fluor de la résine substituée de formule générale (XV) obtenue à l'étape précédente est transformé en fonction amine, de façon à obtenir une résine substituée de formule générale (XVI) : 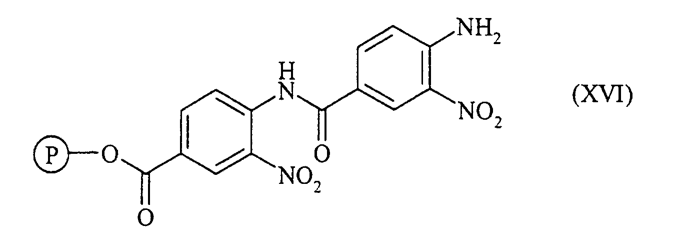 L'amination est opérée selon les méthodes classiques connues de l'homme du métier pour transformer un atome d'halogène en fonction amino, par exemple par substitution nucléophile en opérant en présence d'ammoniac dans un solvant convenable, par exemple le N,N-diméthylformamide.
4) les étapes 2) et 3) telles que décrites ci-dessus sont répétées successivement encore n-2 fois de telle sorte que l'on obtient une résine substituée de formule générale (XVII) : 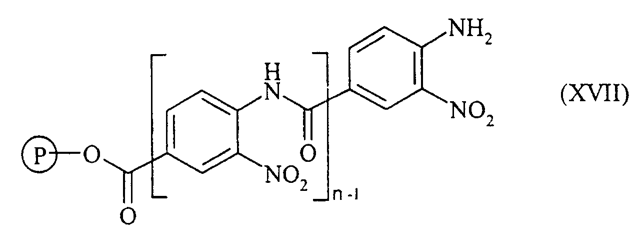
5) On condense ensuite sur la résine substituée de formule générale (XVII) obtenue précédemment un dérivé d'halogénure d'acyle de formule générale (XVIII) : 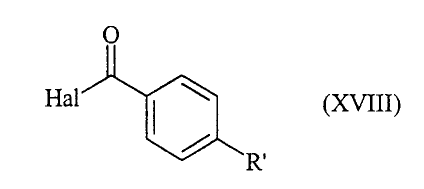 où Hal représente un atome d'halogène choisi parmi le chlore, le brome, l'iode ou le fluor, et R' est tel que défini précédemment,
   afin d'obtenir une résine substituée de formule générale (XIX) : 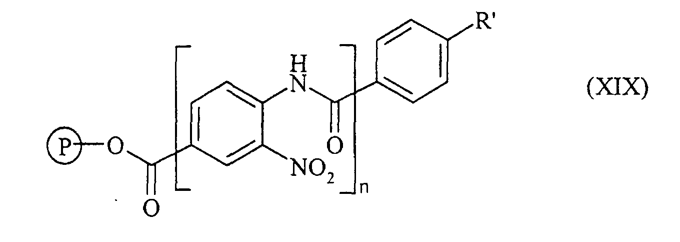 Le couplage est effectué selon les méthodes de couplage peptidique classiques (Bodanski M., Principles and Practices of Peptide Synthesis, Ed. Springe-Verlag) ou par toute méthode analogue connue de l'homme du métier. Notamment, la réaction s'effectue généralement en présence d'une base non-nucléophile dans des solvants aprotiques convenables, à température comprise entre 0 et 100°C, le pH étant ajusté entre 9 et 11.
   A titre d'exemple, le chloroforme, la diméthylformamide, la méthylpyrrolidone, l'acétonitrile, le dichlorométhane, le toluène ou le benzène peuvent être utilisés comme solvant.
   Les bases non-nucléophiles employées sont préférentiellement des amines tertiaires, du carbonate de calcium ou du dicarbonate de sodium. Encore plus préférentiellement, les bases utilisées sont des amines tertiaires comme par exemple la triéthylamine (TEA) ou le N-éthyldiisopropylamine.
   Avantageusement, le couplage peptidique est effectué entre 0 et 50°C, et de préférence entre 10 et 30°C.
   Le dérivé d'halogénure d'acyle de formule générale (XVIII) est soit commercial, soit est obtenu a partir de l'acide correspondant selon toute méthode connue de l'homme du métier pour obtenir un halogénure d'acyle à partir d'un acide. Par exemple, on peut opérer par action du chlorure de thionyle à température comprise entre 70°C et 90°C environ.
   Le dérivé d'acide correspondant est soit commercial soit est obtenu :
   a) par alkylation de l'acide 4-hydroxybenzoïque commercial selon les méthodes classiques connues de l'homme du métier ou selon des méthodes analogues lorsque R' représente un groupe OR₃,
   b) par réduction suivie d'une alkylation de l'acide 4-nitrobenzoïque commercial selon les méthodes classiques connues de l'homme du métier ou selon des méthodes analogues lorsque R' représente un groupe NHR₃ ou, ou bien
   c) par addition nucléophile du dérivé acide COOH-NHR₃ ou COOH-NR₄R₅ sur l'acide 4-hydroxybenzoïque commercial selon les méthodes classiques connues de l'homme du métier ou selon des méthodes analogues lorsque R' représente un groupe - O-CO-NHR₃ ou bien -O-CO-NR₄R₅.
6) La résine substituée de formule générale (XIX) obtenue à l'étape précédente est alors réduite de façon à obtenir une résine substituée par un produit poly-cyclisé de formule générale (XX) : 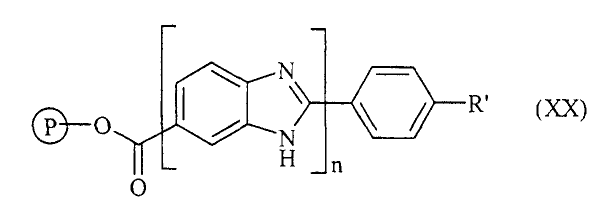 La réduction est effectuée de préférence en présence d'un acide de Lewis dans un solvant convenable. A titre d'acide de Lewis, on utilise par exemple le chlorure d'étain ou le chlorure de chrome. A titre de solvant convenable, on utilise par exemple la N,N-diméthylformamide ou 1a N-méthylpyrrolidinone
7) le produit poly-cyclisé obtenu à l'étape précédente est clivé de la résine et obtient ainsi un dérivé de formule générale (XXI) : 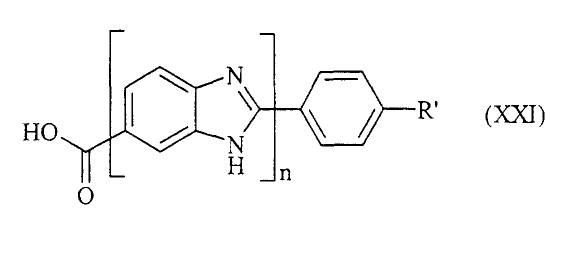 Le clivage de la résine est effectué selon les méthodes classiques connues de l'homme du métier ou selon toute autre méthode analogue. Par exemple, on opère en présence d'acide trifluoroacétique à température comprise entre 10°C et 50°C.
8) Enfin, on obtient les dérivés d'oligobenzimidazole de formule générale (I) selon l'invention à partir du dérivé de formule générale (XXI) obtenu à l'étape précédente, par substitution de la fonction acide par le groupe R, R étant défini comme précédemment, de façon analogue aux méthodes décrites ci-avant en 7) pour la première variante de préparation en phase solide.

Les nouveaux dérivés d'oligobenzimidazole selon la présente invention, ainsi que leurs intermédiaires de synthèse, peuvent être éventuellement purifiés par des méthodes physiques telles que la cristallisation ou la chromatographie.

Par ailleurs, les dérivés lipidiques d' oligobenzimidazole selon l'invention ainsi que leurs intermédiaires peuvent être transformés en sels métalliques ou en sels d'addition avec les bases azotées selon les méthodes connues en soi. Ces sels peuvent être obtenus selon les méthodes habituelles qui n'altèrent pas le reste de la molécule, notamment par action d'une base métallique (par exemple alcaline ou alcalino-terreuse), d'ammoniac ou d'une amine sur un produit cité ci-dessus dans un solvant approprié tel qu'un alcool, un éther ou l'eau, ou par réaction d'échange avec un sel d'un acide organique. Le sel formé précipite après concentration éventuelle de sa solution, il est séparé par filtration, décantation et/ou lyophilisation.

Les dérivés lipidiques d'oligobenzimidazole selon l'invention peuvent être également transformés en sels d'addition avec les acides. Les composés de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropyl-amine, N,N-diméthyléthanolamine, benzylamine, dicyclohexylamine, N-benzyl phénéthylamine, N,N'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates).

Un autre objet de l'invention concerne les compositions comprenant un dérivé d'oligobenzimidazole tel que défini ci-avant et un acide nucléique.

Un autre objet de l'invention concerne les compositions telles que définies précédemment et comprenant en outre un ou plusieurs adjuvants.

A titre d'adjuvant, on peut par exemple citer les co-lipides neutres qui sont capables de s'associer aux complexes formés entre l'ADN et les dérivés d'oligobenzimidazole selon l'invention et d'en améliorer le pouvoir transfectant. En particulier, on peut utiliser des lipides naturels ou synthétiques, zwitterioniques ou dépourvus de charges ioniques dans les conditions physiologiques. Des exemples représentatifs de co-lipides neutres incluent le cholestérol, la dioléylphosphatidyléthanolamine (DOPE), l'oléoylpalmitoylphosphatidyléthanolamine (POPE), les distéaroyl-, dipalmitoyl-, dimirystoylphosphatidyléthanolamines ainsi que leurs dérivés N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM 1 et GM2).

Ces différents co-lipides neutres peuvent être obtenus soit par synthèse, soit par extraction à partir d'organes (comme par exemple le cerveau) ou d'oeufs, par des techniques classiques connues de l'homme du métier. Par exemple, l'extraction des lipides naturels peut être réalisée au moyen de solvants organiques (voir également Biochemistry, Lehninger).

Les compositions selon l'invention comprennent en général 0,01 à 20 d'un co-lipide neutre pour un équivalent d'acide nucléique (en mole/mole), et de préférence 0,05 à 5 équivalents d'un co-lipide neutre.

On peut également utiliser comme adjuvant des composés qui permettent d'améliorer la biodisponibilité, par exemple le polyéthylène glycol.

Selon un autre mode de réalisation, les compositions de la présente invention peuvent en outre contenir un élément de ciblage permettant d'orienter le transfert de l'acide nucléique. Cet élément de ciblage peut être un élément de ciblage extracellulaire permettant d'orienter le transfert de l'ADN vers certains, types cellulaires ou certains tissus souhaités (cellules tumorales, cellules hépatiques, cellules hématopoiétiques...). Il peut également s'agir d'un élément de ciblage intracellulaire permettant d'orienter le transfert de l'acide nucléique vers certains compartiments cellulaires privilégiés (mitochondries, noyau etc...).

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit de sucres, de peptides ou de protéines tels que des anticorps ou des fragments d'anticorps, des ligands de récepteurs cellulaires ou des fragments de ceux-ci, des récepteurs ou des fragments de récepteurs, etc... En particulier, il peut s'agir de ligands de récepteurs de facteurs de croissance, de récepteurs de cytokines, de récepteurs de type lectines cellulaires, ou de ligands à séquence RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalydés tel que le sialyl Lewis X, ou encore un fragment Fab d'anticorps, ou un anticorps simple chaîne (ScFv).

Les quantités respectives de chaque composant peuvent être ajustées aisément par l'homme du métier en fonction du dérivé d'oligobenzimidazole utilisé, de l'acide nucléique, du ou des adjuvants et des applications recherchées (notamment du type de cellules à transfecter).

On entend au sens de l'invention par "acide nucléique" les acides désoxyribonucléiques double brin, formant une double hélice qui comprend un petit sillon et un grand sillon. Il peut s'agir de séquences naturelles ou artificielles, et notamment d'ADN génomique (ADNg), d'ADN complémentaire (ADNc), de séquences hybrides ou de séquences synthétiques ou semi-synthétiques. Ces acides nucléiques peuvent être d'origine humaine, animale, végétale, bactérienne, virale, etc... Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Selon un mode de réalisation particulier, les acides nucléiques sont constitués par des vecteurs, en particulier des vecteurs d'expression, des vecteurs recombinants, des plasmides, des épisomes etc... Lesdits vecteurs comprennent une séquence codante et tous les éléments nécessaires à l'expression de ladite séquence codante, notamment des éléments de régulation de l'expression de l'acide nucléique à insérer, tels que des promoteurs et des séquences activatrices (« enhancers ») ou des séquences d'initiation et d'arrêt de la transcription appropriées, mais aussi d'autres éléments comme par exemple des séquences codant pour une origine de réplication fonctionnelle ou non, des gènes marqueurs, des régions de liaison à d'autres composants cellulaires, des séquences signal, des séquences de polyadénylation etc...

On entend par séquence codante un gène d'intérêt thérapeutique placé en phase avec des séquences de régulation, par exemple un ou plusieurs promoteurs et un terminateur transcriptionnel actifs dans les cellules cibles.

Au sens de l'invention, on entend par gène d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant une stabilité accrue, une activité modifiée, etc... Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc... (FR 2 684 514), les facteurs de croissance, les neurotransmetteurs ou leurs précurseurs ou enzymes de synthèse, les facteurs trophiques (BDNF, CNTF, NGF, IGF, GMF, aFGF, bFGF, NT3, NT5, HARP/pléiotrophine, etc...), les apolipoprotéines (ApoAI ApoAIV, ApoE, etc..., FR 2 704 556), la dystrophine ou une minidystrophine (FR 2 681 786), la protéine CFTR associée à la mucoviscidose, les gènes suppresseurs de tumeurs (p53, Rb, RaplA, DCC, k-rev, etc..., FR 2 704 234), les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme, catabolisme etc...

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique capable de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment contre des micro-organismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... Il peut aussi s'agir de promoteur, inductible ou répressible.

Par ailleurs, l'acide nucléique peut également comporter, en particulier en amont du gène d'intérêt thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, intratrachéale, intrapéritonéale, etc... De préférence, les compositions de l'invention contiennent un véhicule pharmaceutiquement acceptable pour une formulation injectable, notamment pour une injection directe au niveau de l'organe désiré, pour une administration par voie topique (sur peau et/ou muqueuse) ou pour une administration par aérosolisation. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acides nucléiques utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, par exemple au niveau des tumeurs, ou dans les voies circulatoires, soit d'un traitement de cellules en culture suivi de leur réimplantation *in vivo,* par injection ou greffe. Les tissus concernés dans le cadre de la présente invention sont par exemple les muscles, la peau, le cerveau, les poumons, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, les tissus conjonctifs, etc...

L'invention a également pour objet l'utilisation des dérivés d'oligobenzimidazole tels que définis ci-avant pour le transfert de d'acides nucléiques dans les cellules *in vitro, in vivo* ou *ex vivo.* Plus précisément, la présente invention a pour objet l'utilisation des composés tels que définis ci-avant pour la préparation d'un médicament pour le transfert d'acide nucléique dans des cellules. L'acide nucléique contenu dans ledit médicament code un produit protéique ou nucléique, ou constitue ledit produit nucléique, apte à corriger des maladies *in vivo* ou *ex vivo* dans lesquelles ledit produit protéique ou nucléique intervient.

L'invention concerne en outre une méthode de transfert d'acides nucléiques dans les cellules comprenant une première étape au cours de laquelle l'acide nucléique est mis en contact avec au moins un dérivé d'oligobenzimidazole selon l'invention et éventuellement avec un ou des adjuvants et/ou un ou plusieurs véhicules physiologiquement compatible pour former un complexe, et une seconde étape consistant à mettre le complexe ainsi formé en contact des cellules.

La mise en contact des cellules avec le complexe peut être réalisée par incubation des cellules avec ledit complexe (pour des utilisations *in vitro* ou *ex vivo*)*,* ou par injection ou aérosolisation du complexe dans un organisme (pour des utilisations *in vivo*)*.* L'incubation est réalisée de préférence en présence de par exemple de 0,01 à 1000 µg d'acide nucléique pour 10⁶ cellules. Pour une administration *in vivo,* des doses d'acide nucléique allant de 10⁻⁴ à 10 mg peuvent par exemple être utilisées.

Les dérivés d'oligobenzimidazole selon l'invention sont utilisables pour le transfert d'acides nucléiques dans des cellules primaires ou dans des lignées établies. Il peut s'agir de cellules fibroblastiques, musculaires, nerveuses (neurones, astrocytes, cellules gliales), hépatiques, hématopoiétiques (lymphocytes, CD34, dendritiques, etc...), épithéliales etc..., sous forme différenciée ou pluripotente (précurseurs).

Enfin, les utilisations des compositions selon l'invention peuvent concerner aussi bien l'homme que tout animal tel que les ovins, les bovins, les animaux domestiques (chien, chat etc...), les chevaux, les poissons etc...

Outre les dispositions qui précèdent, la présente invention comprend également d'autres caractéristiques et avantages qui ressortiront des exemples et figures qui suivent, et qui doivent être considérés comme illustrant l'invention sans en limiter la portée. Notamment, la demanderesse propose à titre non-limitatif divers protocoles opératoires ainsi que des intermédiaires réactionnels susceptibles d'être mis en oeuvre pour préparer les agents de transfert de formule générale (I). Bien entendu, il est à la portée de l'homme du métier de s'inspirer de ces protocoles ou produits intermédiaires pour mettre au point des procédés analogues en vue de conduire à ces mêmes composés.

### FIGURES

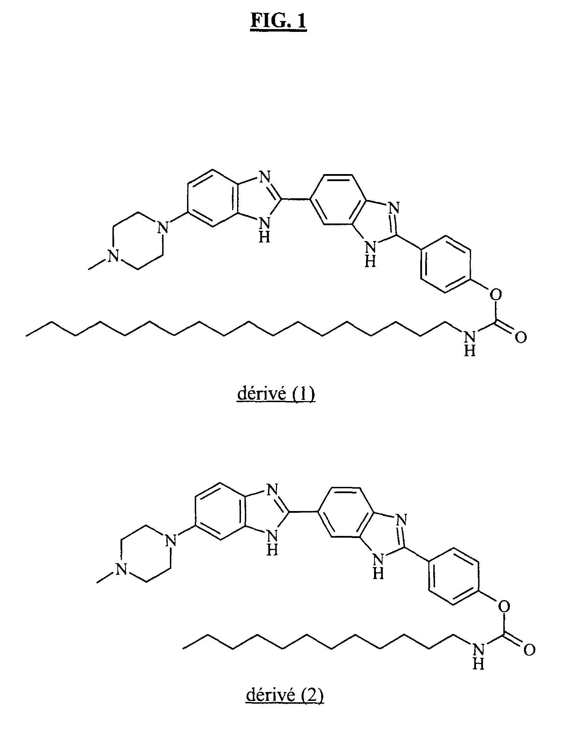

**Figure 1 :** Structure des dérivés d'oligobenzimidazole (1) et (2) dont la préparation est exposée dans les exemples 1 et 2.
**Figure 2 :** Signal d'émission de fluorescence à 450 nm de complexes ADN/dérivé (1) (courbe pleine) et du dérivé (1) seul (courbe en pointillés) en fonction de quantités croissantes de dérivé (1) en nmol/µg d'ADN. La concentration en ADN est de 50 µg/ml.
**Figure 3 :** Signal d'émission de fluorescence à 450 nm de complexes ADN/dérivé (2) (courbe pleine) et du dérivé (2) seul (courbe en pointillés) en fonction de quantités croissantes de dérivé (2) en nmol/µg d'ADN. La concentration en ADN est de 50 µg/ml.
**Figure 4:** Gel d'agarose d'un plasmide d'ADN complexé avec les dérivés (1) et (2), à différentes concentrations de dérivé exprimées en nmol de produit par µg d'ADN.
   « * » : bande jaune caractéristique du dérivé non complexé a l'ADN, et qui reste donc au niveau du point de injection.
   « ** » : bande bleue caractéristique du produit complexé a l'ADN.
**Figure 5** : Gel d'agarose d'un plasmide d'ADN complexé avec les dérivés (1) et (2), à différentes concentrations de dérivé exprimées en nmol de produit par µg d'ADN. Le gel a été révélé sous la même lampe U.V. que pour la figure 4, mais avec du bromure d'éthidium.

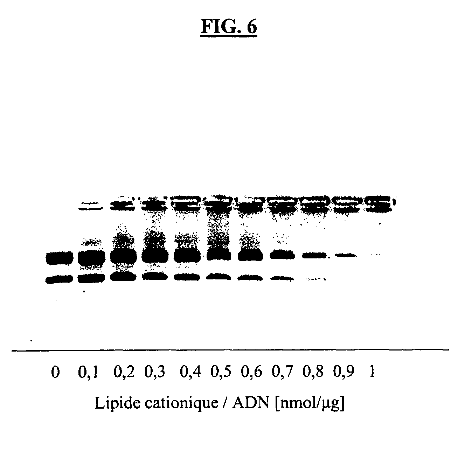

**Figure 6 :** Gel d'agarose (0,8 %) de 1 µg d'un plasmide d'ADN associé à des quantités croissantes d'un lipide cationique de formule : 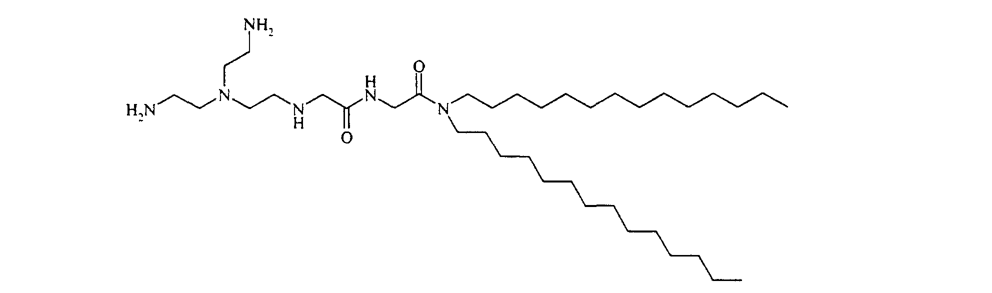 tel que décrit dans la demande de brevet WO 97/18185, les quantités étant exprimée en nmol de lipide cationique par µg d'ADN. Les bandes son révélées avec du bromure d'éthidium et par absorption sous une lampe U.V.
**Figure 7** : Représentation schématique du plasmide pXL3031 utilisé dans les expériences de transfert d'ADN dans les cellules.

### EXEMPLES

Remarque préliminaire : le dodécylisocyanate, l'octadécylisocyanate, la N-éthyldiisopropylamine, le « Hoechst 33258 », la résine de Wang-bromopolystyrène, l'iode, l'iodure de césium, l'acide 3,4-diaminobenzoïque, la 1,2-dianiline, l'acide 3,4-dinitrobenzoïque, le chlorure de thionyle, la pyridine, l'oxyde de cromium, le chlorure de triméthylsilyle, le borohydrure de lithium et le chlorure stanneux sont tous des produits disponibles dans le commerce.

Les spectres RMN (Résonance Magnétique Nucléaire) du proton ont été enregistrés sur des spectromètres Brucker 250 et 400 MHz.

Les analyses CLHP (Chromatographie Liquide haute Performance) ont été réalisées sur un appareil HITACHI équipé d'un autosampler AS-2000A, d'une pompe L-6200A, d'un détecteur UV L 4000 à 220 nm, et d'un intégrateur calculateur D 2500. Le colonne utilisée pour analyser les produits avec des chaînes lipidiques, commercialisée par APPLIED BIOSYSTEMS, est en acier inoxydable de 3 cm de longueur et de 4,6 mm de diamètre. Les phases mobiles sont l'eau et l'acétonitrile additionnées d'acide trifluoroacétique, et la phase stationnaire est l'Aquapore butyl 7 micron. Le débit varie entre 1 et 4 ml/minute. L'autre colonne utilisée pour analyser les produits sans chaînes lipidiques, commercialisée par MERCK, est en acier inoxydable de 25 cm de longueur et de 4,6 mm de diamètre. Les phases mobiles sont l'eau et l'acétonitrile additionnées d'acide trifluoroacétique, et la phase stationnaire est le Lichrospher RP-18 5 micron. Le débit est de 1 ml/minute.

Les Chromatographies sur Couche Mince (CCM) ont été effectuées sur plaque d'aluminium 20x20 recouvertes de gel de silice.

En ce qui concerne les purification CLHP préparatives , l'appareillage utilisé est un ensemble pour la chromatographie en phase liquide en mode gradient, permettant une détection U.V.. Cette chaîne préparative est composée des éléments suivants :
Pompe A : GILSON modèle 305 équipée d'une tête 50 SC.
Pompe B : GILSON modèle 303 équipée d'une tête 50 SC.
Pompe injection : GILSON modèle 303 équipée d'une tête 25 SC.
Module de pression : GILSON modèle 806.
Mélangeur : GILSON modèle 811 C équipé d'une tête de 23 ml.
Détecteur UV : GILSON modèle 119 équipé d'une cellule préparative, et réglé à 220 nm.
Collecteur de fraction : GILSON modèle 202 équipé de portoirs n° 21.
Intégrateur : SHIMADZU modèle C-R6A.
Colonnes : Colonne C4 (10 µ) en acier inoxydable de 25 cm de longueur et de 2,2 cm de diamètre commercialisée par VYDAC modèle 214 TP 1022. Colonne C18 (10 µ) en acier inoxydable de 25 cm de longueur et de 2,2 cm de diamètre commercialisée par VYDAC modèle 218 TP 1022.
La solution de produit à purifier est chargée sur la colonne par la pompe d'injection à un débit de 15 ou 12 ml/minute. Les phases mobiles sont l'eau et l'acétonitrile.

### Exemple 1 : Synthèse du dérivé (1) : 4-[6-(4-méthylpipérazin-1-yl)-1H,3'H-[2,5']bisbenzimidazol-2'-yl]-1-octadécylcarbamoyloxy phényle

0,32 mmol de « Hoechst 33258 » sont dissoutes dans 10 cm³ de diméthylformamide. A cette solution sont ajoutées 2 mmol de N-éthyldiisopropylamine puis 1 mmol d'octadécylisocyanate. Le mélange est agité pendant 24 heures à 50°C et la réaction est suivie par CLHP. On filtre a froid l'urée obtenue sous forme de précipité du fait de l'excès d'isocyanate introduit. On ajoute ensuite de l'acide acétique jusqu'à pH 4, et on évapore le solvant.
Le produit brut obtenu est purifié par CLHP préparative. Les fractions intéressantes sont regroupées et lyophilisées.
On obtient 0,125 mmol de produit salifié soit un rendement de 39,2 %.
CLHP : Rt = 9,81 min.
Spectre de R.M.N. ¹H (400 MHz, (CD₃)₂SO d6, δ en ppm) : 0,86 (t, J = 7 Hz : 3H) ; de 1,15 à 1,40 (mt : 30H) ; 1,51 (mt : 2H) ; 2,92 (s : 3H) ; de 3,00 à 3,15 (mt : 2H) ; 3,10 (mt : 2H) ; 3,26 (mt : 2H) ; 3,61 (d large, J = 10 Hz : 2H) ; 3,91 (d large, J = 10 Hz : 2H) ; 7,20 (s large : 1H) ; 7,25 (d large, J = 9 Hz : 1H) ; 7,36 (d, J = 9 Hz : 2H) ; 7,68 (d, J = 9 Hz : 1H) ; de 7,80 à 7,90 (mt : 2H) ; 8,06 (dd, J = 9 et 1,5 Hz : 1H) ; 8,25 (d, J = 9 Hz : 2H) ; 8,45 (s large : 1H) ; de 9,70 à 9,90 (mf : 1H).

### Exemple 2 : Synthèse du dérivé (2) : 4-(6-(4-méthylpipérazin-1-yl)-1H,3'H-[2,5']bisbenzimidazol-2'-yl]-1-dodécylcarbamoyloxy phényle

0,32 mmol de « Hoechst 33258 » sont dissoutes dans 10 cm³ de diméthylformamide. A cette solution sont ajoutées 2 mmol de N-éthyldiisopropylamine puis 1 mmol de dodécylisocyanate. Le mélange est agité pendant 24 heures à 50°C et la réaction est suivie par CLHP. On filtre a froid l'urée obtenue sous forme de précipité du fait de l'excès d'isocyanate introduit. On ajoute ensuite de l'acide acétique jusqu'à pH 4, et on évapore le solvant.
Le produit brut obtenu est purifié par CLHP préparative. Les fractions intéressantes sont regroupées et lyophilisées.
On obtient 0,134 mmol de produit salifié soit un rendement de 41,9 %.
CLHP : Rt = 11,34 min.
Spectre de RMN ¹ H (400 MHz, (CD₃)₂SO d6, δ en ppm) : 0,89 (t, J = 7 Hz : 3H) ; de 1,25 à 1,45 (mt : 18H) ; 1,56 (mt : 2H) ; 2,93 (s : 3H) ; 3,15 (mt : 2H) ; de 3,30 à 4,20 (mt : 8H) ; 7,17 (dd, J = 9 et 2 Hz : 1H) ; 7,22 (d, J = 2 Hz : 1H) ; 7,34 (d, J = 8,5 Hz : 2H) ; de 7,45 à 7,60 (mf : 1H) ; 7,63 (d, J = 9 Hz : 1H) ; 7,81 (d, J = 8 Hz : 1H) ; 8,06 (d large, J = 8 Hz : 1H) ; 8,24 (d, J = 8,5 Hz : 2H) ; 8,43 (s large : 1H).

### Exemple 3 : Mise en évidence de la formation de complexes entre le dérivé (1) ou (2) et l'ADN par mesure directe de fluorescence

Cet exemple illustre la propriété des dérivés d'oligobenzimidazole selon l'invention à former des complexes avec l'ADN.

Pour cela, la fluorescence de mélange d'ADN avec des quantités croissantes de dérivé (1) ou (2) a été mesurée par excitation à 350 nm et détection à 450 nm.

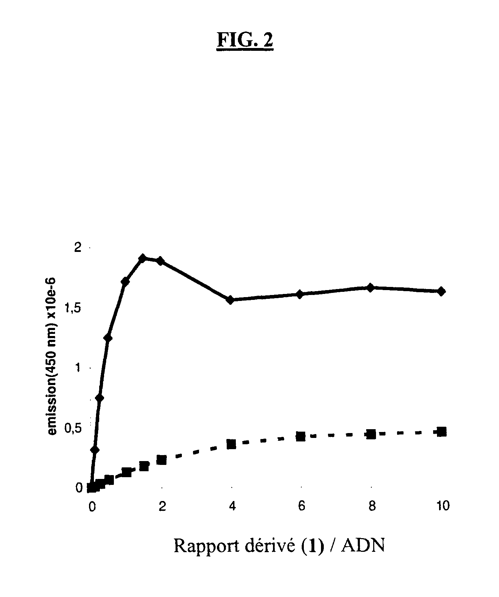

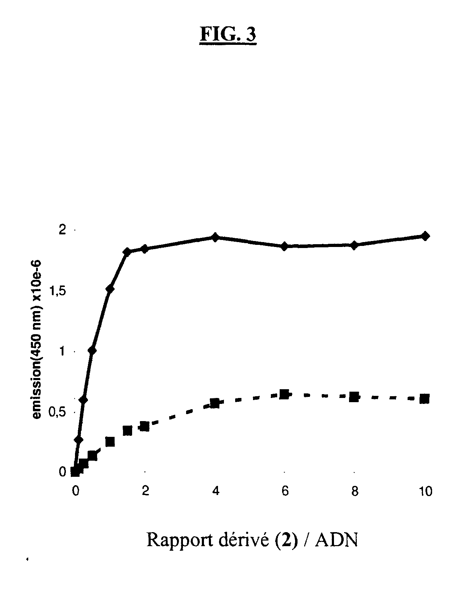

Les résultats sont représentés à la figure 2 pour la formation de complexes avec le dérivé (1) et à la figure 3 pour la formation de complexes avec le dérivé (2).

Dans tous les cas, on constate que lorsqu'il n'y a pas de dérivé d'oligobenzimidazole (1) ou (2) et que l'ADN est seul en solution, aucune fluorescence n'est détectée. Puis, la fluorescence augmente avec des quantités croissantes de dérivé (1) ou (2) jusqu'à atteindre un palier. Cette fluorescence n'est pas identique à la fluorescence émise par le dérivé (1) ou (2) seul, mais en revanche, les courbes obtenues pour les complexes ADN / dérivé présentent un spectre d'émission et d'excitation similaire à celui obtenu pour le « Hoechst 33258 » (résultat non montré).

Ainsi, ces résultats montrent que les dérivés (1) et (2) forment des complexes avec l'ADN et que la saturation du sillon de l'ADN (concentration en dérivé par rapport à la quantité d'ADN au delà de laquelle plus aucun complexe ne se forme) se situe à un rapport dérivé d'oligobenzimidazole/ADN d'environ 1,5-2 nmol/µg.

### Exemple 4 : Etude par électrophorèse d'un gel d'agarose et comparaison avec un lipide cationique de l'art antérieur

Cet exemple complète l'exemple 3 car il met en évidence la formation de complexes d'ADN et de dérivés d'oligobenzimidazole selon l'invention. En outre, cet exemple illustre les propriétés spécifiques de ces complexes ADN/dérivés d'oligobenzimidazole par rapport aux complexes ADN/lipide cationique de l'art antérieur.

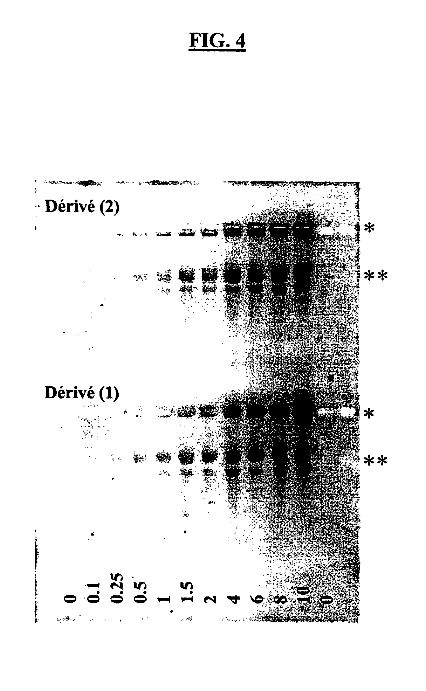

Pour cela, un gel d'agarose d'un plasmide d'ADN mélangé avec des quantités croissantes de dérivé (1) ou (2) selon l'invention a été effectué (voir figure 4). Ce gel a été directement observé sous la lumière d'une lampe U.V. sans être révélé. Deux bandes ont alors pu être observées grâce aux propriétés d'absorption spectrales spécifiques des dérivés selon la présente invention :
- une bande jaune caractéristique du dérivé seul, c'est-à-dire non complexé a l'ADN (bande marquée d'une « * ») : on constate que le dérivé reste dans le point d'injection,
- une bande bleue caractéristique du dérivé complexé a l'ADN (bande marquée d'une « ** »).

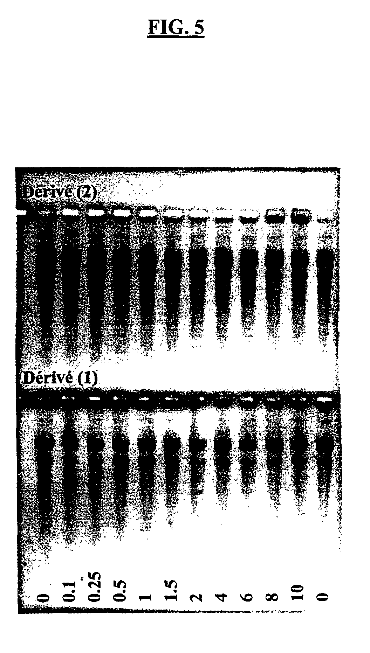

Le même gel d'agarose a ensuite été révélé avec du bromure d'éthydium (voir figure 5). On constate que l'ADN migre de façon identique à l'ADN nu et quelle que soit la concentration en dérivé selon l'invention. Cet exemple illustre donc le fait que les dérivés (1) et (2) donnent des complexes avec l'ADN qui ont les mêmes propriétés de mobilité sous électrophorèse que l'ADN nu, alors que ce n'est pas le cas lorsque l'ADN est complexé avec des lipides cationiques classiques. En effet, la figure 6 montre un gel d'agarose effectué avec des complexes contenant des quantités croissantes d'un lipide cationique : on observe une migration des complexes formés qui varie avec la quantité de lipide cationique présente avec l'ADN. Ce résultat indique que plus la quantité de lipide cationique est augmentée, plus l'ADN est compacté, et moins il migre sur le gel.

Ainsi, les dérivés d'oligobenzimidazole selon la présente invention sont des complexants de l'ADN qui, au contraire des lipides cationiques classiquement utilisés en transfection non-virale de gènes, ne compactent pas l'ADN. Les propriétés de mobilité de l'ADN sont donc conservées, même lorsque des quantités importantes de dérivés selon la présente invention sont ajoutées à l'ADN pour former des complexes. Cette propriété est particulièrement intéressante dans une optique de transfection non-virale de gènes car il serait ainsi possible grâce aux dérivés d'oligobenzimidazole selon la présente invention de former des complexes avec l'ADN permettant la protection dudit ADN vis-à-vis des endonucléases sans pour autant modifier ses propriétés de mobilité.

En outre, il est ainsi également possible de former des complexes qui sont détectables par des méthodes directes, notamment sans révélation par le bromure d'éthydium, grâce aux propriétés de fluorescence spécifiques des dérivés selon la présente invention.

### Exemple 5 : Transfection in vitro de matériel génétique complexé au dérivé (2) selon l'invention en présence et en l'absence de sérum

### A. MATÉRIEL GÉNÉTIQUE UTILISÉ

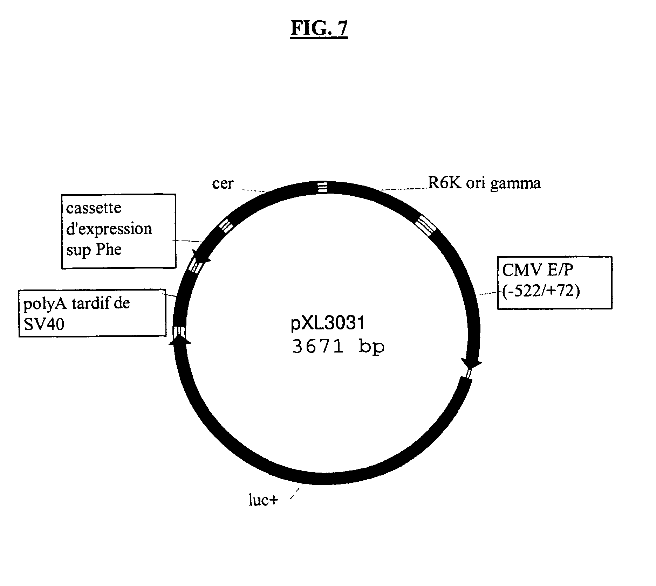

Le plasmide utilisé L'ADN utilisé est le plasmide pXL3031 (voir figure 7) en solution dans un mélange de dextrose 5% et de chlorure de sodium 10 mM à une concentration de 0,5 mg/ml ou de 1,0 mg/ml. Ce plasmide contient le gène luc codant pour la luciférase sous contrôle du promoteur P/E CMV du cytomégalovirus. Sa taille est de 3671 bp. Le plasmide pXL3031 a été purifié selon les méthodes décrites dans la demande de brevet WO 97/35002.
Les solutions d'acide nucléique sont diluées à 20 µg/ml dans du sérum physiologique (chlorure de sodium 0,15 M).

### B. SOLUTIONS CYTOFECTANTES (préparation extemporanée)

Le dérivé (2) d'oligobenzimidazole selon l'invention est mis en solution dans l'eau à concentration variant de 40 à 160 µmol et mélangé volume à volume avec la solution d'ADN. La concentration saline finale est de 75 mmol.

### C. TRANSFECTION

Les cellules HeLa sont cultivées dans les conditions appropriées en microplaques de 24 puits (2 cm²/puits) et sont transfectées alors qu'elles sont en phase exponentielle de croissance et à 50-70 % de la confluence.

Les cellules sont lavées par 2 fois 0,5 cm³ de milieu dépourvu de protéines sériques et remises en croissance soit en milieu sans sérum (transfection en absence de sérum), soit en milieu complet (transfection en présence de sérum). 0,05 cm³ de mélange cytofectant (0,5 µg d'ADN/puits) sont ajoutés aux cellules (3 puits/condition vecteur-ADN). Quand les cellules sont transfectées en l'absence de sérum, le milieu de croissance est supplémenté 2 heures après la transfection par la quantité appropriée de sérum.

L'efficacité de transfection est évaluée à 48 heures post-transfection par une mesure de l'expression de la luciférase selon les recommandations données pour l'utilisation du kit Promega (« Luciférase Assay System »). La toxicité des mélanges cytofectants est estimée par une mesure des concentrations protéiques dans les lysats cellulaires.

Les résultats d'activité luciférase *in vitro* rapportés aux protéines exprimés en RLU/5µl/10s/µg de protéine (écrit plus simplement RLU/µg de protéine, « RLU » signifiant « Unité de Lumière Relative ») sont rassemblés dans le tableau suivant :

| **Concentration** (nmol/µg d'ADN) | 0 | 2 | 4 | 8 | 10 |
|---|---|---|---|---|---|
| **dérivé (2) / ADN** | nd | nd | 1,3 E+03 | 1,7 E+03 | 1,7 E+03 |
| **dérivé (2) / ADN +sérum** | ^{nd} | ^{nd} | 9,8 E+02 | 4,6 E+02 | 1,1 E+02 |
| « nd » signifie « expression non-détectable ». | | | | | |

Les résultats obtenus indiquent qu'il est possible d'obtenir une expression après transfert de matériel génétique complexé à des dérivés selon la présente invention dans des *cellules in vitro,* que ce soit dans un milieu avec ou sans sérum..

### Exemple 6 : Transfection in vivo de matériel génétique complexé avec le dérivé (1) selon l'invention avec ou sans électrotranfert

Le plasmide utilisé est le même que celui décrit précédemment pour l'exemple 5 (pXL3031). De même, les solutions cytofectantes sont préparées de la même façon que dans l'exemple 5.

### TRANSFECTION

25 µl de solutions de complexes dérivé (1)/ADN sont injectées par voie intramusculaire à des souris C57B16, à raison de 4 µg d'ADN / muscle de souris.

L'efficacité de transfection est évaluée à 7 jours post-transfection par une mesure de l'expression de la luciférase selon les recommandations données pour l'utilisation du kit Promega (Luciférase Assay System). Les muscles sont broyés dans 1,5 ml de tampon de lyse (avec inhibiteurs de protéases). Après dosage de l'activité luciférase sur 10 µl, les résultats sont exprimés en RLU/10µl/10sec.

Les résultats d'activité luciférase *in vivo* dans les muscles de souris, exprimés en RLU/10µl/10sec, sont rassemblés dans le tableau suivant :

| | | | | |
|---|---|---|---|---|
| **Concentration en dérivé (1) en nmol/µg d'ADN)** | 0 | 0,2 | 0,5 | 1 |
| **transfection in *vivo* sans électrotransfert** | 9,25 E+04 | 3,45 E+04 | 1,67 E+04 | 1,06 E+04 |
| **transfection in *vivo* avec éléctrotransfert** | 2,96 E+07 | 1,14 E+07 | 2,19 E+07 | 1,82 E+07 |

Les résultats obtenus indiquent qu'il est possible d'obtenir une expression *in vivo* dans le muscle après transfert de matériel génétique complexé à des dérivés selon la présente invention que ce soit en utilisant ou non la technique d'électrotransfert telle que décrite dans les demandes de brevet WO99/01157 et WO 99/01158.

## Revendications

1. Dérivés d'oligobenzimidazole de formule générale (I) : 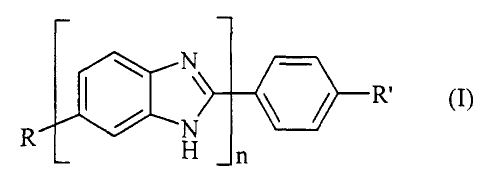 dans laquelle
- R représente un atome d'hydrogène, un radical carboxyle, alkyloxycarbonyle, carbamoyle, alkylcarbamoyle, un groupe pipérazinyle éventuellement substitué en position -4 par un alkyle contenant 1 à 4 atomes de carbone droit ou ramifié, ou bien R représente un groupe imidazolyle,
- n est un entier égal à 2, 3, 4 ou 5, et
- R' représente un groupe -O-R₃, -S-R₃, NHR₃, ou bien -O-CO-NH-R₃ et R₃ représente un groupe alkyle,
- ou bien R' représente un groupe -NR₄R₅ ou bien -O-CO-NR₄R₅ et R₄ et R₅, identiques ou différents, représentent chacun un groupe alkyle,
les radicaux alkyles mentionnés ci-dessus étant, sauf spécification contraire, droits ou ramifiés, éventuellement saturés, et contenant 12 à 22 atomes de carbone, étant entendu que R' est différent de OR₃ avec R₃ représentant un substituant dodécyle lorsque R représente le méthyl-4 pipérazinyle et que n est égal à 2, ainsi que leurs sels métalliques, leurs sels d'addition avec les bases azotées et leurs sels d'addition avec les acides.

2. Dérivés d'oligobenzimidazole selon la revendication 1 de formule générale (II) : 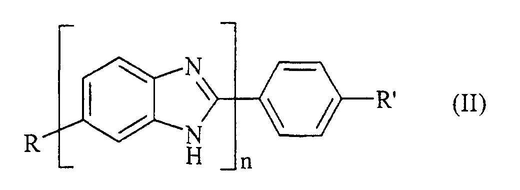 dans laquelle
- R représente un atome d'hydrogène ou un groupe pipérazinyle éventuellement substitué en position -4 par un alkyle contenant 1 à 4 atomes de carbone droit ou ramifié,
- n est un entier égal à 2 ou 3, et
- R' représente un groupe -OR₃ , NHR₃ ou -O-CO-NH-R₃ et R₃ représente un groupe alkyle,
- ou bien R' représente un groupe NR₄R₅ ou -O-CO-NR₄R₅ et R₄ et R₅, identiques ou différents, représentent chacun un groupe alkyle, les radicaux alkyles mentionnés ci-dessus étant, sauf spécification contraire, droits ou ramifiés, éventuellement saturés, et contenant 12 à 22 atomes de carbone,
étant entendu que R' est différent de OR₃ avec R₃ représentant un substituant dodécyle lorsque R représente le méthyl-4 pipérazinyle et que n est égal à 2,
ainsi que leurs sels métalliques, leurs sels d'addition avec les bases azotées et leurs sels d'addition avec les acides.

3. Dérivés d'oligobenzimidazole selon la revendication 1 **caractérisés en ce qu'**il s'agit du 4-[6-(4-méthylpipérazin-1-yl)-1H,3 'H-[2,5']bisbenzimidazol-2'-yl]-1-octadécyl-carbamoyloxy phényle (dérivé (1)) ou du 4-[6-(4-méthylpipérazin-1-yl)-1H,3'H-[2,5']bisbenzimidazol-2'-yl]-1-dodécylcarbamoyloxy phényle (dérivé (2)).

4. Composition **caractérisée en ce qu'**elle comprend un dérivé d'oligobenzimidazole tel que défini dans la revendication 1, 2 ou 3 ou le dérivé pour lequel R' représente un groupe OR₃ avec R₃ représentant un substituant dodécyle, R représente le méthyl-4 pipérazinyle et n est égal à 2, et un acide nucléique.

5. Composition selon la revendication 4 **caractérisée en ce qu'**elle comprend en outre un ou plusieurs adjuvants.

6. Compositions selon la revendication 4 ou 5 **caractérisée en ce qu'**elle contient en outre un véhicule pharmaceutiquement acceptable pour une formulation injectable, topique ou sous forme d'aérosol.

7. Utilisation d'un dérivé d'oligobenzimidazole tel que défini dans la revendication 1, 2 ou 3 ou du dérivé pour lequel R' représente un groupe OR₃ avec R₃ représentant un substituant dodécyle, R représente le méthyl-4 pipérazinyle et n est égal à 2, pour le transfert d'acides nucléiques dans des cellules *in vitro.*

8. Utilisation d'un dérivé d'oligobenzimidazole tel que défini dans la revendication 1, 2 ou 3 ou du dérivé pour lequel R' représente un groupe OR₃ avec R₃ représentant un substituant dodécyle, R représente le méthyl-4 pipérazinyle et n est égal à 2, pour la préparation d'un médicament pour le transfert d'acide nucléique dans des cellules.

9. Méthode de transfert d'acides nucléiques dans des cellules *in vitro* **caractérisée en ce qu'**elle comprend une première étape au cours de laquelle l'acide nucléique est mis en contact avec au moins un dérivé d'oligobenzimidazole tel que défini dans la revendication 1, 2 ou 3 ou avec le dérivé pour lequel R' représente un groupe OR₃ avec R₃ représentant un substituant dodécyle, R représente le méthyl-4 pipérazinyle et n est égal à 2, et éventuellement avec un ou des adjuvants et/ou un ou plusieurs véhicules physiologiquement compatibles pour former un complexe, et une seconde étape qui consiste à mettre le complexe ainsi formé en contact des cellules.

10. Composition selon la revendication 4, 5 ou 6 pour son utilisation comme médicament.

## Claims

1. Oligobenzimidazole derivatives of general formula (I): 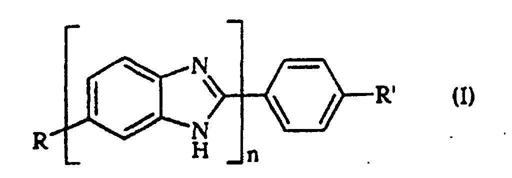 in which
- R represents a hydrogen atom, a carboxyl, alkoxycarbonyl, carbamoyl or alkylcarbamoyl radical or a piperazinyl group optionally substituted in position
- 4 with an alkyl containing 1 to 4 straight or branched carbon atoms, or alternatively R represents an imidazolyl group,
- n is an integer equal to 2, 3, 4 or 5, and
- R' represents a group -O-R₃, -S-R₃, NHR₃ or
- O-CO-NH-R₃ and R₃ represents an alkyl group,
- or alternatively R' represents a group -NR₄R₅ or -O-CO-NR₄R₅ and R₄ and R₅, which may be identical or different, each represent an alkyl group, the alkyl radicals mentioned above being, except where specified otherwise, straight or branched, optionally saturated and containing 12 to 22 carbon atoms,
it being understood that R' is other than OR₃ with R₃ representing a dodecyl substituent when R represents 4-methylpiperazinyl and that n is equal to 2, as well as the metal salts thereof, the addition salts thereof with the nitrogenous bases and the addition salts thereof with acids.

2. Oligobenzimidazole derivatives according to Claim 1, of general formula (II): 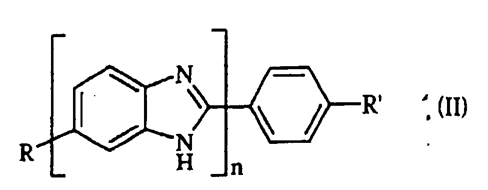 in which
- R represents a hydrogen atom or a piperazinyl group optionally substituted in position -4 with an alkyl containing 1 to 4 straight or branched carbon atoms,
- n is an integer equal to 2 or 3, and
- R' represents a group -OR₃, NHR₃ or -O-CO-NH-R₃ and R₃ represents an alkyl group,
- or alternatively R' represents a group NR₄R₅ or -O-CO-NR₄R₅ and R₄ and R₅, which may be identical or different, each represent an alkyl group, the alkyl radicals mentioned above being, except where otherwise specified, straight or branched, optionally saturated and containing 12 to 22 carbon atoms,
it being understood that R' is other than OR₃ with R₃ representing a dodecyl substituent when R represents 4-methylpiperazinyl and that n is equal to 2,
as well as the metal salts thereof, the addition salts thereof with the nitrogenous bases and the addition salts thereof with acids.

3. Oligobenzimidazole derivatives according to Claim 1, **characterized in that** the derivative is 4-[6-(4-methyl-1-piperazinyl)-2H,3'H-[2,5']bisbenzimidazol-2'-yl]-1-octadecylcarbamoyloxy phenyl (derivative (1)) or 4-[6-(4-methyl-1-piperazinyl)-1H,3'H-[2,5']bisbenzimidazol-2'-yl]-1-dodecylcarbamoyloxy phenyl (derivative (2)).

4. Composition, **characterized in that** it comprises an oligobenzimidazole derivative as defined in Claim 1, 2 or 3 or the derivative for which R' represents a group OR₃ with R₃ representing a dodecyl substituent, R represents 4-methylpiperazinyl and n is equal to 2, and a nucleic acid.

5. Composition according to Claim 4, **characterized in that** it also comprises one or more adjuvants.

6. Composition according to Claim 4 or 5, **characterized in that** it also contains a vehicle which is pharmaceutically acceptable for an injectable or topical formulation or for a formulation in the form of an aerosol.

7. Use of an oligobenzimidazole derivative as defined in Claim 1, 2 or 3 or of the derivative for which R' represents a group OR₃ with R₃ representing a dodecyl substituent, R represents 4-methylpiperazinyl and n is equal to 2, for the transfer of nucleic acids into cells *in vitro.*

8. Use of an oligobenzimidazole derivative as defined in Claim 1, 2 or 3 or of the derivative for which R' represents a group OR₃ with R₃ representing a dodecyl substituent, R represents 4-methylpiperazinyl and n is equal to 2, for the preparation of a medicinal product for transferring nucleic acid into cells.

9. Method for transferring nucleic acids into cells *in vitro,* **characterized in that** it comprises a first step during which the nucleic acid is placed in contact with at least one oligobenzimidazole derivative as defined in Claim 1, 2 or 3 or with the derivative for which R' represents a group OR₃ with R₃ representing a dodecyl substituent, R represents 4-methylpiperazinyl and n is equal to 2, and optionally with one or more adjuvants and/or one or more physiologically compatible vehicles to form a complex, and a second step which consists in placing the complex thus formed in contact with the cells.

10. Composition according to Claim 4, 5 or 6, for its use as a medicinal product.

## Patentansprüche

1. Oligobenzimidazol-Derivate der allgemeinen Formel (I): 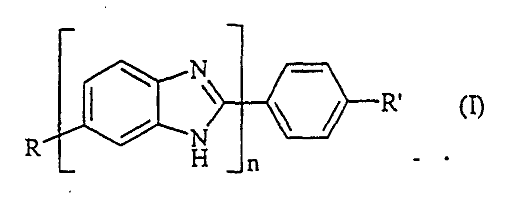 worin
- R ein Wasserstoffatom, einen Carboxyl-, Alkoxycarbonyl-, Carbamoyl-, Alkylcarbamoyl-Rest, eine Piperazinylgruppe, die gegebenenfalls in Position 4 durch ein geradkettiges oder verzweigtes Alkyl, das 1 bis 4 Kohlenstoffatome enthält, substituiert ist, darstellt oder R eine Imidazolylgruppe darstellt;
- n eine ganze Zahl, 2, 3, 4 oder 5, ist und
- R' eine Gruppe -O-R₃, -S-R₃, NHR₃ oder auch - O-CO-NH-R₃ darstellt und R₃ eine Alkylgruppe darstellt;
- oder R' eine Gruppe -NR₄R₅ oder auch -OCO-NR₄R₅ darstellt und R₄ und R₅, die gleich oder unterschiedlich sind, jeweils eine Alkylgruppe darstellen,
wobei die oben genannten Alkylreste, außer bei. gegenteiliger Spezifizierung, geradkettig oder verzweigt sind, gegebenenfalls gesättigt sind und 12 bis 22 Kohlenstoffatome enthalten; mit der Maßgabe, dass R' von OR₃, worin R₃ einen Dodecylsubstituenten darstellt, verschieden ist, wenn R 4-Methylpiperazinyl ist, und n gleich 2 ist, sowie ihre Metallsalze, ihre Additionssalze mit Stickstoffbasen und ihre Additionssalze mit Säuren.

2. Oligobenzimidazol-Derivate nach Anspruch 1 mit der allgemeinen Formel (II) 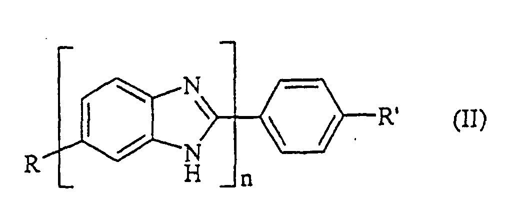 worin
- R ein Wasserstoffatom oder eine Piperazinylgruppe, die gegebenenfalls in Position 4 durch ein geradkettiges oder verzweigtes Alkyl, das 1 bis 4 Kohlenstoffatome enthält, substituiert ist, darstellt;
- n eine ganze Zahl, 2 oder 3, ist, und
- R' eine Gruppe -OR₃, NHR₃ oder -O-CO-NH-R₃ darstellt und R₃ eine Alkylgruppe darstellt;
- oder R' eine Gruppe NR₄R₅ oder -O-CO-NR₄R₅ darstellt und R₄ und R₅, die gleich oder unterschiedlich sind, jeweils eine Alkylgruppe darstellen;
wobei die oben genannten Alkylreste, außer bei gegenteiliger Spezifizierung, geradkettig oder verzweigt sind, gegebenenfalls gesättigt sind und 12 bis 22 Kohlenstoffatome enthalten; mit der Maßgabe, dass R' von OR₃, worin R₃ einen Dodecylsubstituenten darstellt, verschieden ist, wenn R 4-Methylpiperazinyl darstellt und n 2 ist; sowie ihre Metallsalze, ihre Additionssalze mit Stickstoffbasen und ihre Additionssalze mit Säuren.

3. Oligobenzimidazolderivate nach Anspruch 1, **dadurch gekennzeichnet, dass** es sich um 4-[6-(4-Methylpiperazin-1-yl)-1H,3'H-[2,5']bisbenzimidazol-2'-yl]-1-octadecylcarbamoyloxy-phenyl (Derivat (1)) oder 4-[6-(4-Methylpiperazin-1-yl)-1H,3'H-[2,5]bisbenzimidazol-2'yl]-1-dodecylcarbamoyloxy-phenyl (Derivat (2)) handelt.

4. Zusammensetzung, **dadurch gekennzeichnet, dass** sie ein Oligobenzimidazolderivat, wie es in Anspruch 1, 2 oder 3 definiert ist oder das Derivat, für das R' eine OR₃-Gruppe darstellt, worin R₃ einen Dodecylsubstituenten darstellt, R 4-Methylpiperazinyl darstellt und n 2 ist, und eine Nucleinsäure umfasst.

5. Zusammensetzung nach Anspruch 4, **dadurch gekennzeichnet, dass** sie außerdem ein Adjuvans oder mehrere Adjuvantien umfasst.

6. Zusammensetzung nach Anspruch 4 oder 5, **dadurch gekennzeichnet, dass** sie außerdem ein Vehikel umfasst, das für eine injizierbare Formulierung eine topische Formulierung oder eine Aerosolform annehmbar ist.

7. Verwendung eines Oligobenzimidazolderivats, wie es in Anspruch 1, 2 oder 3 definiert ist, oder des Derivats, für das R' eine OR₃-Gruppe, worin R₃ einen Dodecylsubstituenten darstellt, bedeutet, R 4-Methylpiperazinyl darstellt und n gleich 2 ist, zur Transfektion von Nucleinsäuren in Zellen in vitro.

8. Verwendung eines Oligobenzimidazolderivats, wie es in Anspruch 1, 2 oder 3 definiert ist, oder eines Derivats, für das R' eine OR₃-Gruppe, worin R₃ einen Dodecylsubstituenten darstellt, bedeutet, R 4-Methylpiperazinyl darstellt und n 2 ist, zur Herstellung eines Medikaments zur Transfektion von Nucleinsäure in Zellen.

9. Verfahren zur Transfektion von Nucleinsäuren in Zellen in vitro, **dadurch gekennzeichnet, dass** es eine erste Stufe umfasst, im Verlauf der die Nucleinsäure mit mindestens einem Oligobenzimidazolderivat, wie es in Anspruch 1, 2 oder 3 definiert ist, oder mit dem Derivat, in dem R' eine OR₃-Gruppe, worin R₃ einen Dodecylsubstituenten darstellt, bedeutet, R 4-Methylpiperazinyl darstellt und n gleich 2 ist, und gegebenenfalls mit einem Adjuvans oder Adjuvantien und/oder einem oder mehreren Vehikeln, die zur Bildung eines Komplexes physiologisch kompatibel sind, in Kontakt gebracht wird, und eine zweite Stufe, die darin besteht, den so gebildeten Komplex mit den Zellen in Kontakt zu bringen, umfasst.

10. Zusammensetzung nach Anspruch 4, 5 oder 6 zur Verwendung als Medikament.
